# EUROPEAN PATENT SPECIFICATION

(11) **EP 0 622 050 B1**
(45) Date of publication and mention of the grant of the patent: **05.03.1997**
(21) Application number: 94106605.2
(22) Date of filing: 27.04.1994
(51) Int. Cl.: A61B 17/32

(54) **Microsurgical scalpel assembly**
Mikrochirurgisches Skalpel
Bistouri microchirurgical

(30) Priority: 30.04.1993 US 54174
(43) Date of publication of application: 02.11.1994
(73) Proprietor: EAGLE VISION INC., Memphis, Tennessee 38119 (US)
(72) Inventor: Webb, Nicholas J., Wrightwood, CA 92397 (US); Mendius, Richard W., Memphis, TN 3818-0614 (US)
(74) Representative: Grünecker, Kinkeldey, Stockmair & Schwanhäusser Anwaltssozietät

(56) References cited:
- CH-A- 490 072
- DE-U- 8 703 525
- US-A- 739 371
- US-A- 3 706 106
- US-A- 3 793 726
- US-A- 3 906 626
- US-A- 4 071 952
- US-A- 4 165 745
- US-A- 4 499 898
- US-A- 4 552 146
- US-A- 4 576 164
- US-A- 4 635 914
- US-A- 4 674 500
- US-A- 4 719 915
- US-A- 4 735 202
- US-A- 4 759 363
- US-A- 4 825 545
- US-A- 4 903 390
- US-A- 5 026 386
- US-A- 5 059 210
- US-A- 5 092 852
- US-A- 5 222 951
- US-A- 5 292 329

## Description

### BACKGROUND OF THE INVENTION

This invention relates to a microsurgical scalpel assembly according to the preamble of claim 1. More particularly this invention is directed to a microsurgical scalpel assembly with enhanced safety and useability characteristics for an operating surgeon and a concomitant capability of being economically utilized as a high precision instrument.

In the surgical scalpel industry and particularly the microsurgical scalpel arena at least two problems are becoming of increasing concern. One level of concern surrounds the economic utilization of surgical instruments in the medical community. In this connection, low cost scalpels have been envisioned which are essentially disposable, however, initial point sharpness and cutting edge quality less than at which exists in more expensive, high precision instruments. However, with increasing economic pressures being placed upon the medical community, tradeoffs are being seriously evaluated and low cost disposable scalpels are being utilized as opposed to more high quality precision instruments.

In addition to instrument quality and an attendant potential for less than optimum surgical results the medical community has a growing concern with respect to body fluid transmitted diseases. In this, acquired immune deficiency syndrome (AIDS), Hepatitis B, etc., are becoming prevalent enough such that health care professionals are increasingly concerned about maintaining an uncompromising isolation integrity with respect to the body fluid of patients. Although wearing disposable rubber gloves is a precaution of preference, and even in certain instances wearing double gloves is an accepted practice, when utilizing a sharply pointed and/or sharp bladed surgical instrument such as microsurgical scalpels, puncture wounds, even though a double gloved surgeon, is not only conceivable but almost inescapable. It has been estimated that as much as two percent of sharp product handling results in accidental stabs in the medical community.

The difficulty suggested in the proceeding are not intended to be exhaustive but rather are among many which may tend to limit the effectiveness of prior microsurgical scalpels. Other noteworthy problems may also exist; however, those presented above should be sufficient to demonstrate that microsurgical scalpels appearing in the past will admit to worthwhile improvement.

The US-A-4,735,202 which discloses the features of the preamble of claim 1, shows a disposable micro-surgical knife having a blade guard that can be locked into a blade covering position and then easily removed from the knife body prior to introduction to the sterile field.

### OBJECTS AND ADVANTAGES OF THE INVENTION

It is, therefore, a general object of the invention to provide a novel microsurgical scalpel assembly which will obviate or minimize problems of the type previously described.

It is an advantage of the invention to provide a microsurgical high quality scalpel which may be advantageously re-sterilized and reused in a microsurgical procedure.

It is a further advantage of the invention to provide a microsurgical scalpel assembly wherein sharp point and cutting edge integrity of high quality scalpels may be faciley maintained during handling and autoclaving re-sterilization procedures in the presence of other instruments.

It is another advantage of the invention to provide a microsurgical scalpel assembly wherein the tendency of a surgeon to receive a puncture wound during handling is minimized.

It is a further advantage of the invention to provide a microsurgical scalpel assembly wherein blade shapes and lengths of a large variety may be accommodated while concomitantly providing isolation integrity of the blade for a surgeon during handling and permitting re-sterilization without jeopardizing the sharpness integrity of the microsurgical scalpel.

### Brief Summary of A Preferred Embodiment of the Invention

A preferred embodiment of the invention as specified in claim 1 is intended to accomplish at least some of the foregoing advantages includes an elongate scalpel handle and a scalpel blade affixed at one end of the scalpel handle and extending away from the handle. A shield is releasably affixed to the one end of the scalpel handle and extends away from the handle to operably surround the scalpel blade to isolate the blade from puncture contact with a surgeon during handling. The shield further operably isolates a tip and cutting edge of the microsurgical blade during an autoclaving operation wherein other instruments may be positioned in close physical proximity. Further, the shield is provided with a plurality of elongate apertures which are positioned adjacent the scalpel blade and thus permit circulation and full sterilization during an autoclaving operation.

### THE DRAWINGS

Other objects and advantages of the present invention will become apparent from the following detailed description of preferred embodiments taken in conjunction with the accompanying drawings wherein:
FIG. 1 is an axonometric view of a microsurgical scalpel assembly in accordance with a preferred embodiment of the invention.
FIG. 2 is an exploded axonometric view of a microsurgical scalpel assembly as depicted in FIG. 1 wherein a scalpel blade shield is disclosed in a position to be mounted about the scalpel blade by being slid from the base of the scalpel to and surrounding the blade end of the scalpel;
FIG. 3 is a axonometric detailed view of a scalpel blade shield in accordance with a preferred embodiment of the invention;
FIG. 4 is a cross-section view of the microsurgical scalpel shield depicted in FIG. 3.
FIG. 5 is a cross-sectional view taken along section line 5-5 in FIG. 4;
FIG. 6 is an end view of the microsurgical scalpel shield depicted in FIG. 4;
FIG. 7 is an end view of the microsurgical scalpel shield depicted in FIG. 5;
FIG. 8 is a side elevational view of a microsurgical scalpel in accordance with a preferred embodiment of the invention;
FIG. 9 is a cross-sectional view taken along section line 9-9 in FIG. 8;
FIG. 10 is a cross-sectional view taken along section line 10-10 in FIG. 8;
FIG. 11 is a cross-sectional view taken along section line 11-11 in FIG. 8;
FIG. 13 is an axonometric view of a microsurgical scalpel assembly in accordance with still another preferred embodiment of the invention;
FIG. 14 is an exploded axonometric view of a microsurgical scalpel assembly as depicted in FIG. 13;
FIG. 15 is a plan view of a microsurgical shield in accordance with the embodiment of the invention depicted in FIGs. 13 and 14;
FIG. 16 is an end view of the microsurgical shield as depicted in FIG. 15;
FIGs. 17 a-c disclose a sequence of views of a surgeon applying a microsurgical scalpel shield to a microsurgical scalpel in accordance with the subject invention; and
FIGs. 18 a-c disclose a corresponding sequence of removing a microsurgical scalpel shield from a scalpel in accordance with the subject invention.

### DETAILED DESCRIPTION

### Illustrative Structural Embodiments

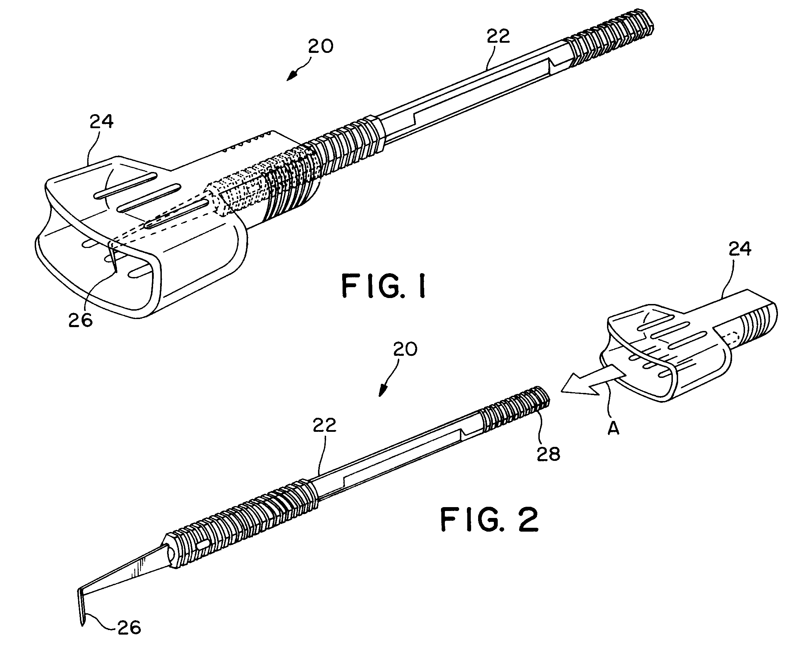

Turning to FIG. 1 there will be seen an axonometric scalpel assembly 20 in accordance with a preferred embodiment of the invention. In this, the assembly includes a generally elongate scalpel handle 22 and a scalpel shield 24 which is releasably affixed to one end of the scalpel handle. It will be noted that the shield extends away from the scalpel handle and is operable to isolate a scalpel blade 26 from contact by a physician during handling of the microsurgical scalpel assembly. The blade depicted is intended to be illustrative and other shaped blades such as arrow blades for phacoemulsification incisions and placement of intraocular lenses, radial keratotomy blades, crescent, circular and general dissection blades are all contemplated by the subject invention.

Turning now specifically to FIG. 2, it will be seen that the microsurgical scalpel assembly 20 is depicted in an expanded condition wherein the microsurgical shield 24 is directed toward a base end 28 of the scalpel handle 22 in the direction of arrow A. The shield 24, as will be discussed in detail presently hereafter, is designed to be slid from the base of the surgical instrument to the sharp blade end and thus avoid any likelihood or possibility that a surgeon will need to thread a sharp blade into a shield and thus create a possibility of a stab wound during an inattentive or hurried motion.

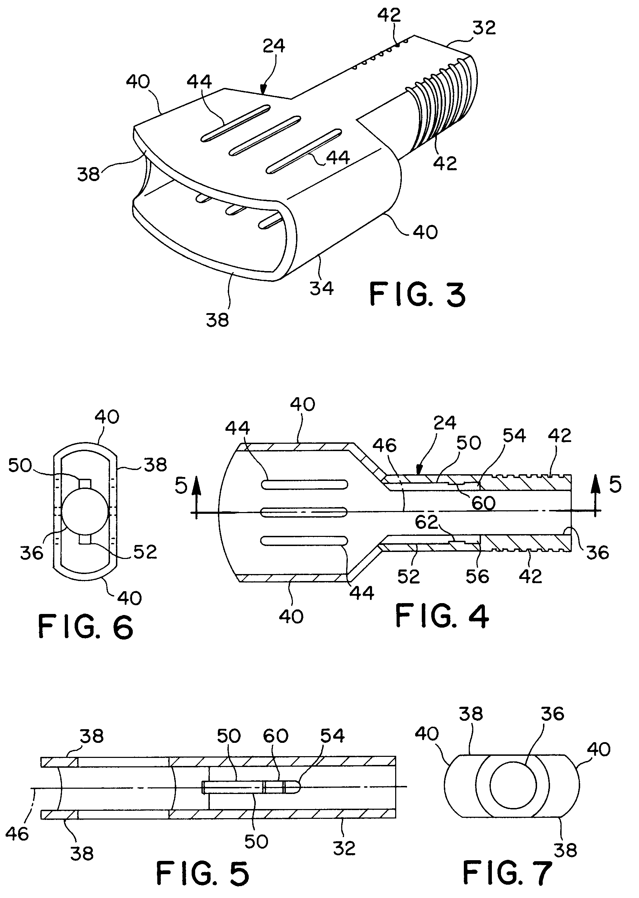

Referring now to FIGs. 3-7 there will be seen various detailed views of a preferred embodiment of a microsurgical scalpel shield in accordance with the subject invention. The microsurgical shield includes a base portion 32 and a hood portion 34. The base portion comprises a generally elongate cylinder having an axial bore 36, note FIGs. 4, and 6-7, which is circular in cross section and coaxially extends through the generally cylindrical base member. The hood 34 is expanded outwardly from the base member and is generally or essentially rectangular in cross-sectional configuration, note FIG. 6. the hood 34 includes a pair of mutually opposing long sidewall surfaces 38 and short sidewall surfaces 40. The sidewalls 38 are generally planar in configuration while the shorter end walls 40 assume a gentle arcuate configuration as depicted in FIGs. 6 and 7.

The base portion 32 exteriorally includes a plurality of mutually parallel and circumferentially extending grooves 42 which operably extend about the base and serve to facilitate grasping by an operating physician in a manner which will be discussed below.

The sidewall surfaces 38 of the hood portion includes a plurality of apertures 44 which are fashioned as elongate slots and extend through the hood portion of the general direction of a central longitudinal axis 46 of the microsurgical shield. The elongate slots 44 operably are dimensioned and positioned to be adjacent a cutting blade, a piercing and/or cutting blade 26 of a microsurgical scalpel, note again FIG. 1, and facilitate full re-sterilization during an autoclaving procedure by insuring full circulation around and about the microsurgical blade even though the blade is protected by the shield 24.

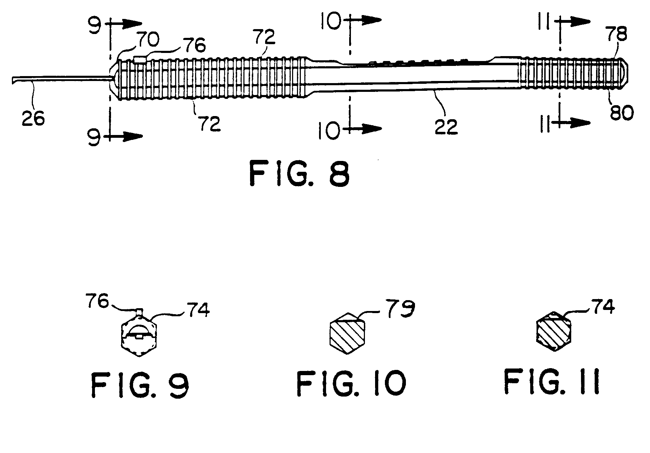

The bore 36 of the microsurgical shield 24 is interially fashioned with longitudinally extending slots 50 and 52. A base portion of the slots terminate in an abutting stop 54 and 56 respectively and each slot is fashioned with a raised land segment 60 and 62 as depicted in FIG. 4. Function of the slots is to engage a compatibly dimensioned tab in a microsurgical scalpel as particularly depicted in FIG. 8-11.

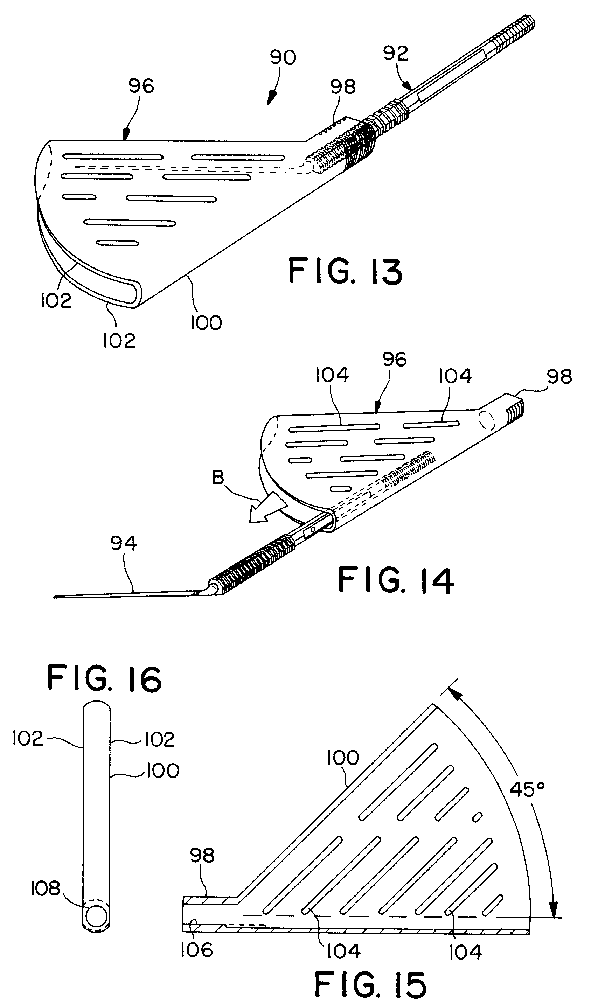

Turning to those figures, FIG. 8 discloses a side elevational view of an elongate scalpel handle 22 as previously shown in FIG. 1. The handle 22, note particularly FIGs. 10 and 11 is a generally solid member having a hexagonal cross-sectional configuration. At one end 70 of the scalpel handle, an illustrative microsurgical blade 26 is releasably attached in a manner known to those of ordinary skill in the art and thus not presented in detail herein. The subject invention includes the provision of a combination of a plurality of circumferential and mutually parallel grooves 72 at the one end 70 of the scalpel which extend about the scalpel as depicted particularly in FIG. 8. An external hexagon configuration of the scalpel, note particularly FIG. 9, having side surfaces 74. This combination provides peripheral grooves 72 to permit the scalpel to be faciley manipulated in an axial direction by a surgeon while the hexagonal shape facilitates translational and rotational manipulation of the scalpel as desired during a surgical procedure. As shown in FIG. 9, an upstanding tab 76 or boss is integrally projected from the other end 70 of the scalpel handle and operably cooperates with and releasably engages one or the other of the elongate slots 50 and 52 as depicted in FIG. 4 during an assembly operation.

As shown in FIGs. 8 and 10, a mid portion of the scalpel 22 may be reduced by the creation of a land area 79 operable to receive indicia to identify the scalpel as desired.

A base end or other end 78 of the scalpel is also advantageously configured with a plurality of mutually parallel circumferential grooves or slots 80. In addition the base includes a hexagonal configuration having sides 74 which are extensions from the first end. The combination of circumferential grooves and hexagonal land surfaces enable the instrument to be faciley manipulated during surgery and for receiving the microsurgical shield in a manner to be discussed below.

Turning now to FIGs. 13-16, there will be seen yet another preferred embodiment of the invention wherein a microsurgical scalpel assembly 90 is depicted of a type operable or myringotomy procedues. A scalpel handle 92 carries an elongate surgical blade 94 of a type suitable for ear and throat surgery. Typically myringotomy blade lengths may be up to three inches and from 0 to 30 degrees of angulation with respect to a scalpel handle. The detail of the handle 92 is similar in all substantial respects with that depicted in FIGs. 8-11 and will not be repeated.

The microsurgical scalpel assembly 90 includes a shield structure 96 having a base portion 98 and a hood portion 100. The hood portion in the subject embodiment is fashioned in a general fan shape configuration to accommodate the elongate myringotomy surgical blade 94. The fan shaped configuration has in one embodiment a span of approximately 45 degrees, note FIG. 15. Generally flat opposing sidewall surfaces 102 as shown in FIGs. 13 and 16 are closed at the edges with arcuate side walls. The flat sidewall surfaces 102 are operably fashioned with a plurality of elongate apertures 104 which are designed to enhance autoclave re-sterilization of the blade 94 by permitting the free access and flow around the blade during an autoclaving procedure. A base portion 98 of the microsurgical shield includes an axial bore 106 in a manner previously discussed with respect to the embodiment of FIGs. 3-7 including at least one internal slot 108 which is operable to receive a corresponding tab, not shown, from a microsurgical scalpel 92 in a manner and position as previously illustrated in connection with FIG. 8. Accordingly in operation, the shield 96 is pushed over the base end of the scalpel 92 in the direction of arrow B toward the scalpel blade to cover the blade to snap into position and to operably cover the blade during a handling operation while concomitantly permitting full re-sterilization during an autoclaving procedure.

In each of the above embodiments of the invention, the hood is preferably fabricated from a clear polysulfone which is a high temperature polymer having structural rigidity and high temperature resistance to permit autoclave resterilization procedures.

While ophthalmology and otology scalpels have been depicted, the subject invention operable for use for other surgical grade scalpels where similar problems have been encountered.

### Sequence of Utilization

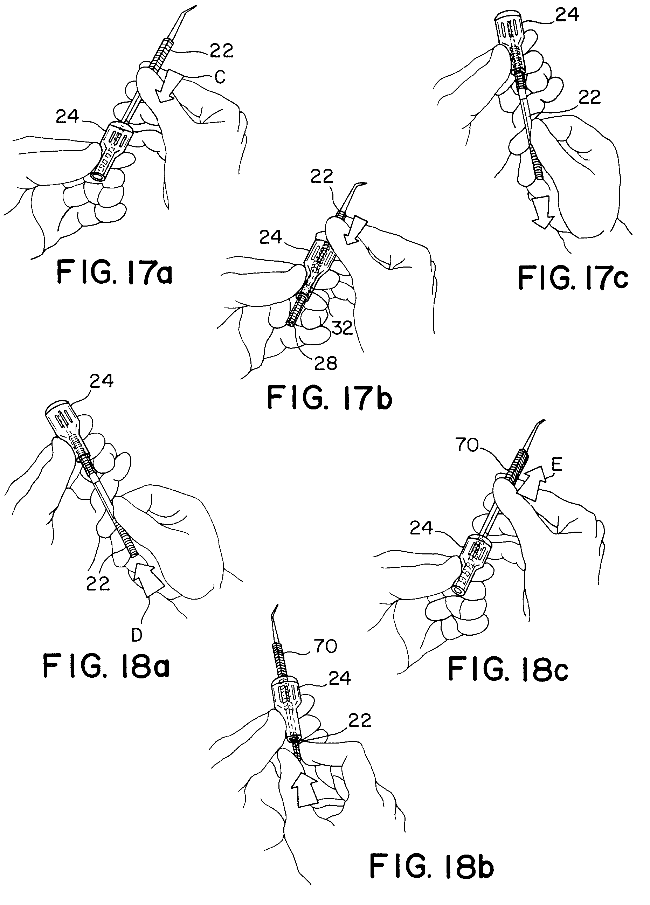

Referring specifically to FIGs. 17a-c there will be depicted a sequence of views showing a surgeons hands and a microsurgical shield to be applied to a microsurgical scalpel to form an assembly in accordance with the subject invention. More particularly, and as shown in FIG. 17a, a surgeon's left hand is shown holding a microsurgical shield 24 at its base by the circumferential grooves and the base end of the scalpel 22 is guided into the axial bore through the shield 24 as shown generally by directional arrow C. In FIGs. 17b, the surgeon is retaining his grasp on both instruments and a base 28 of the scalpel 22 projects through a base portion 32 of the microsurgical shield 24. Still further movement of the scalpel is effected by transferring the surgeons hand such that the right hand now grasps a mid portion of the scalpel handle and pulls a forward end of the scalpel into engagement wherein a tab 76 of the scalpel handle, note again FIG. 8, is slid into and engaged with a corresponding slot 50 or 52 within the shield 24, note particularly FIGs. 4 and 6.

In brief sequence, the scalpel is inserted into the shield with the knife blade pointing away from the surgeon and the base of the handle is guided into the large or expanded end of the microsurgical shield. In FIG. 17b, the handle is telescoped through the base or small portion of the microsurgical shield. As depicted in FIG. 17c, holding the microsurgical protective shield firmly in the left hand, the right hand is transferred to the base of the scalpel and the scalpel handle is pulled through until it clicks into a stop on the shield in a protective position around the blade.

A reverse or removal sequence is depicted in FIGs. 17a-c. In this sequence, a surgeon operably grasps the base of the shield 24 with a left hand and pushes in the direction of arrow D the handle of the surgical scalpel, note particularly FIG. 18a. Further axial pushing is depicted in FIG. 18b until a forward end 70 of the scalpel 22 extends beyond the shield 24. At this time a surgeon may transfer his right hand to grasping the forward end 70 of the scalpel and remove the scalpel from the shield 24 in the direction of arrow E.

In sequence the removal process includes grasping the subject microsurgical scalpel assembly with the knife blade pointing away from the surgeon and firmly holding the microsurgical shield with the left hand while pushing the handle through the shield in the direction of the blade end as depicted in arrow D. Once enough handle is exposed as shown in FIG. 18b, a surgeon may transfer his right hand to a forward position on the handle as depicted in FIG. 18c and remove the surgical device without providing an opportunity for cutting or a puncture of any of the surgeon's fingers by a tip portion of the scalpel.

### Summary of Major Advantages of the Invention

After reading and understanding the foregoing description of various embodiments of the subject microsurgical scalpel assembly, in conjunction with the drawings, it will be appreciated that several distinct advantages of the subject invention are obtained. One major advantage is the provision of a microsurgical shield in cooperation with a scalpel to isolate a scalpel blade 26 from the hands of a surgeon during a handling operation. Assembly and disassembly of the subject invention is provided from a base end of the unit such that a surgeon is not required to guide or align a sharp surgical tip adjacent his own hands.

The enlarged microsurgical scalpel shield 24, 82 and 96, accommodate angulated offsets of conventional scalpel blades and a plurality of apertures permit full and complete re-sterilization of the blade during an autoclaving operation within a protective shield or hood.

The microsurgical scalpel handle is provided with a plurality of circumferential grooves near one end adjacent a scalpel blade and at an opposite end to facilitate handling, including assembly and disassembly of a microsurgical scalpel hood. An interior slot within the hood and a corresponding tab 76 upon the surgical handle permits the hood to be snap fit into position so that it may be faciley maintained during all handling operations.

The shields 24, 82 and 96 operably protect the point and cutting surfaces of high quality surgical instruments and thus a scalpel of fine grade and sharpness may be repetitively used and re-sterilized without becoming dull from inadvertent touching, bumping or laying against other surgical instruments which can take the fine edge off cutting surfaces.

In describing the invention, reference has been made to preferred embodiments and illustrative advantages of the invention. Those skilled in the art, however, after reading the subject disclosure, may recognize additions, deletions, modifications and/or other changes which will fall within the purview of the subject invention as defined in the claims.

## Claims

1. A microsurgical scalpel assembly (20, 90) comprising:
an elongate scalpel handle (22, 92) having a longitudinal axis whereby the handle (22, 92) may be grasped by a surgeon and rotated about its longitudinal axis;
a scalpel blade (26, 94) affixed at one end (70) of said scalpel handle (22, 92) terminating with at least one operative cutting edge; and
a shield (24, 96) releasably connected to said one end (70) of said scalpel handle (22, 92) and extending away from said one end of said scalpel handle (22, 92) to operably surround said scalpel blade (26, 94) when the shield (24, 96) is installed on said one end (70) of said scalpel, said shield (24, 96) is designed to be slid from the base (28) to the one end (70) of said scalpel handle (22, 92) to be connected to the one end (70) of said scalpel handle (22, 92), wherein said shield (24, 96) comprises:
a base portion (32, 98) operably to releasably engage said one end (70) of said scalpel handle (22, 92), said base portion (32, 98) having an elongate bore operable to slidingly receive the other end (70) of said elongate scalpel handle (22, 92) and to releasably engage said one end (70) of said scalpel handle (22, 92); and
a hood portion (34, 100) connected to said base portion, (32, 98) and further comprising a circumferential edge which defines an opening of the hood portion (34, 100);
said elongate scalpel handle (22, 92) having a laterally extending tab (76) radially projecting away from said elongate scalpel handle (22, 92) generally at said one end (70) thereof, **characterized in that** said scalpel blade (26, 94) extends away from said scalpel handle (22, 92) at an angle with respect to said longitudinal axis of said scalpel handle (22, 92), both the longitudinal axis of said tab (76) and said scalpel blade (26, 94) are generally in the same plane, said hood portion (34, 100) has an enlarged configuration with respect to said base portion (32, 98) and is spaced from and substantially completely surrounding said scalpel blade (22, 92) when said shield (24, 96) is in an installed position, said hood portion (34, 100) comprising mutually opposing long side wall surfaces (38, 100) and mutually opposing short side wall surfaces (40) so as to appear substantially rectangular at any cross-section taken along a line normal to a central longitudinal axis of said scalpel handle (22, 92) and
the total width of the tab (76) and scalpel handle (22, 92) together as measured perpendicular to the axis of the scalpel handle (22, 92) is longer than the inner parallel distance between the long side wall surfaces (38, 102) and shorter than the inner parallel distance between the short side walls surfaces (40) of the hood portion (34, 100).

2. A microsurgical scalpel assembly as defined in claim 1 wherein:
said long side wall surfaces (38, 100) are generally planar and said hood portion further comprising a throat portion adjacent the base portion (32, 98) of the shield, said throat portion defined in part by a segment of said mutually opposed short side wall surfaces (40) which radial converge toward the bore (36, 106) of the base portion (32, 98) of said shield (24, 96);

3. A microsurgical scalpel assembly (20, 90) as defined in claim 2 wherein:
said segment of said mutually opposed short side wall surfaces (40) which define in part said throat portion of said hood (34, 100) are outwardly arcuate.

4. A microsurgical scalpel assembly (20, 90) as defined in claim 2 wherein:
said mutually opposed short side wall surfaces (40) of said hood portion (34, 100) are outwardly arcuate.

5. A microsurgical scalpel assembly (20, 90) as defined in claim 1 wherein said shield (24, 96) further comprising:
at least one elongate slot (50, 108) fashioned within said elongate bore (36, 106) and being dimensioned to cooperate with said tab (76) of said elongate scalpel handle (22, 92), said elongate slot (50, 108) terminating in a stop (56) to limit relative motion between said shield (24, 96) and said elongate scalpel handle (22, 92).

6. A microsurgical scalpel assembly (20, 90) as defined in claim 1 wherein:
said hood portion (34, 100) is provided with a plurality of apertures (44, 104) fashioned through said hood portion (34, 100) to operably facilitate sterilization of said scalpel blade (26, 94) affixed at said one end of said microsurgical scalpel assembly (20, 90).

7. A microsurgical scalpel assembly (20, 90) as defined in claim 5 wherein said plurality of apertures (44, 104) comprise:
mutually parallel elongate slots fashioned through said shield (24, 96) in an operative location of said shield adjacent to said scalpel blade (26, 94) and having central longitudinal axes which are mutually parallel with longitudinal axis of said scalpel handle (22, 92).

8. A microsurgical scalpel assembly (20, 90) as defined in claim 1 wherein said elongate scalpel handle (22, 92) comprises:
an elongate member which is essentially hexagonal in cross-section and having a plurality of mutually parallel, circumferential grooves (72) fashioned at said one end (70) of said scalpel handle (22, 92) such that axial movement of the said microsurgical scalpel is facilitated by said parallel, circumferential grooves (72) and rotational and lateral movement of the scalpel is facilitated by said hexagonal configuration of said scalpel handle (22, 92).

9. A microsurgical scalpel assembly (20, 90) as defined in claim 5 and further comprising:
a raised land portion (62) positioned within said at least one elongate slot (52, 108) adjacent to, but axially spaced from, said stop (56) such that said tab (76) extending from said scalpel handle (22, 92) operably slides through said elongate slot (52, 108) and over said land to (62) click into a resting retained position between said land (62) and said stop (56).

10. A microsurgical scalpel assembly (20, 90) as defined in claim 5 wherein said at least one elongate slot (52) comprises:
a pair of mutually opposing elongate slots (52) positioned within said bore (36) upon opposite sides of the base (32) of said shield (24, 96).

11. A microsurgical scalpel assembly (20, 90) as defined in claim 5 wherein said elongate scalpel handle (22, 92) further comprises:
a plurality of mutually parallel, circumferential grooves (80) fashioned about the other end (78) of said scalpel handle (22, 92) to facilitate handling of the scalpel handle (22, 92) by a physician to initially align and engage the shield (24, 96) with the base (78) of the scalpel handle (22, 92) and thereafter slide the shield (24, 96) from the base (78) of the scalpel handle (22, 92) to the end (70) of the handle (22, 92) holding said scalpel blade (26, 94).

12. A microsurgical scalpel assembly (20, 90) as defined in claim 1, wherein the area of each sequential cross-section increasing at each location further away from said base portion (78) such that said hood portion (34, 100) exhibits a fanned out configuration to accommodate an extended and angulated configuration of said scalpel blade (26, 94) with respect to said elongate scalpel handle (26, 94).

## Patentansprüche

1. Mikrochirurgisches Skalpell (20, 90) mit:
einem länglichen Skalpellgriffstück (22, 92) mit einer Längsachse, wobei das Griffstück (22, 92) von einem Chirurg um seine Längsachse ergriffen werden kann;
eine Skalpellschneide (26, 94), die an einem Ende (70) des Skalpellgriffstücks (22, 92) angebracht ist und in mindestens einer Arbeitsschneidkante endet; und
eine Abdeckung (24, 96), die lösbar an einem Ende (70) des Skalpellgriffstücks (22, 92) befestigt ist und sich weg von dem einen Ende des Skalpellgriffstücks (22, 92) erstreckt, um die Skalpellschneide (26, 94) zu umgeben, wenn die Abdeckung (24, 96) an einem Ende (70) des Skalpells angeordnet ist, wobei die Abdeckung (24, 96) geformt ist, um von dem Basisende (28) zu dem einen Ende (70) des Skalpellgriffstücks (22, 92) geschoben zu werden und mit dem einen Ende des Skalpellgriffstücks (22, 92) verbunden zu werden, worin die Abdeckung (24, 96) umfaßt:
einen Basisbereich (36, 98), der lösbar mit dem einen Ende (70) des Skalpellgriffstücks (22, 92) in Verbindung steht, wobei der Basisbereich (32, 98) eine Längsbohrung aufweist, die verschiebbar das andere Ende (70) des länglichen Skalpellgriffstücks (22, 92) aufnimmt, und lösbar mit dem einen Ende (70) des Skalpellgriffstücks (22, 92) in Eingriff steht; und
einen Haubenbereich (34, 100), der mit dem Basisbereich (32, 98) verbunden ist und weiter eine umlaufende Kante aufweist, die eine Öffnung des Haubenbereichs (34, 100) umgrenzt;
das längliche Skalpellgriffstück (22, 92) weist einen seitlich sich erstreckenden Vorsprung (76) auf, der radial von dem länglichen Skalpellgriffstück (22, 92) im wesentlichen an dessen einen Ende (70) hervorsteht,
**dadurch gekennzeichnet**,
daß sich die Skalpellschneide (26, 94) von dem Skalpellgriffstück (22, 92) in einem Winkel in Bezug zur Längsachse des Skalpellgriffstücks (22, 92) wegerstreckt, wobei sowohl die Längsachse des Vorsprungs (76) als auch die Skalpellschneide (26, 94) im wesentlichen in der selben Ebene angeordnet sind, wobei der Haubenbereich (34, 100) eine größere Ausbildung als der Basisbereich (32, 98) aufweist und von der Skalpellschneide (22, 92) beabstandet ist und diese im wesentlichen vollständig umgibt, wenn sich die Abdeckung (24, 96) in einer aufgeschobenen Stellung befindet, wobei der Haubenbereich (34, 100) gegenüberliegende lange Seitenwandflächen (38, 102) und gegenüberliegende kurze Seitenwandflächen (40) aufweist, um im wesentlichen in jedem Querschnitt rechtwinklig zu erscheinen, der entlang einer Linie senkrecht zu einer mittigen Längsachse des Skalpellgriffstücks (22, 92) geschnitten wird; und
die Gesamtbreite des Vorsprungs (76) und des Skalpellgriffstücks (22, 92) zusammen, wenn sie rechtwinklig zu der Achse des Skalpellgriffstücks (22, 92) gemessen wird, länger ist als der innere parallele Abstand zwischen den langen Seitenwandflächen (38, 102) und kürzer ist als der innere parallele Abstand zwischen den kurzen Seitenwandflächen (40) des Haubenbereichs (34, 100).

2. Mikrochirurgisches Skalpell nach Anspruch 1, worin:
die langen Seitenwandflächen (38, 102) im wesentlichen eben sind und der Haubenbereich weiter einen Halsbereich angrenzend des Basisbereichs (32, 98) der Abdeckung aufweist, wobei der Halsbereich teilweise durch ein Segment der gegenüberliegenden kurzen Seitenwandflächen (40) gebildet ist, welche in Richtung der Bohrung (36, 106) des Basisbereichs (32, 98) der Abdeckung (24, 96) radial zulaufen.

3. Mikrochirurgisches Skalpell (20, 90) nach Anspruch 2, worin:
das Segment der gegenüberliegenden kurzen Seitenwandflächen (40), welches teilweise den Halsbereich der Haube (34, 100) bildet, nach außen gebogen ist.

4. Mikrochirurgisches Skalpell (20, 90) nach Anspruch 2, worin:
die gegenüberliegenden kurzen Seitenwandflächen (40) des Haubenbereichs (34, 100) nach außen gebogen sind.

5. Mikrochirurgisches Skalpell (20, 90) nach Anspruch 1, worin die Abdeckung (24, 96) weiter umfaßt:
mindestens einen Längsschlitz (50, 108), der in der Längsbohrung (36, 106) angeordnet ist und dimensioniert ist, um mit dem Vorsprung (76) des länglichen Skalpellgriffstücks (22, 92) in Eingriff zu stehen, wobei der Längsschlitz (50, 108) mit einem Anschlag (56) endet, um die Relativbewegung zwischen der Abdeckung (24, 96) und dem länglichen Skalpellgriffstück (22, 92) zu begrenzen.

6. Mikrochirurgisches Skalpell (20, 90) nach Anspruch 1, worin der Haubenbereich (34, 100) mit mehreren Öffnungen (44, 104) versehen ist, die sich durch den Haubenbereich (34, 100) erstrecken, um eine Sterilisation der Skalpellschneide (26, 94), die an dem einen Ende des mikrochirurgischen Skalpells (20, 90) angeordnet ist, zu erleichtern.

7. Mikrochirurgisches Skalpell (20, 90) nach Anspruch 5, worin die mehreren Öffnungen (44, 104) umfassen:
parallele Längsschlitze, die sich durch die Abdeckung (24, 96) in eine betriebsgünstige Stelle der Abdeckung angrenzend der Skalpellschneide (26, 94) erstrecken und eine mittige Längsachse aufweisen, welche parallel zu der Längsachse des Skalpellgriffstücks (22, 92) angeordnet ist.

8. Mikrochirurgisches Skalpell (20, 90) nach Anspruch 1, worin das längliche Skalpellgriffstück (22, 92) umfaßt:
ein längliches Element, das einen im wesentlichen hexagonalen Querschnitt aufweist, und mehrere parallele Umfangsnuten (72) aufweist, die an dem einen Ende (70) des Skalpellgriffstücks (22, 92) derart angeordnet sind, daß eine Axialbewegung des mikrochirurgischen Skalpells durch die parallelen Umfangsnuten (72) erleichtert ist und eine Dreh- und Seitenbewegung des Skalpells durch die hexagonale Ausbildung des Skalpellgriffstücks (22, 92) begünstigt ist.

9. Mikrochirurgisches Skalpell (20, 90) nach Anspruch 5, das weiterhin umfaßt:
einen erhöhten Bereich (62), der in dem mindestens einen Längsschlitz (52, 108) angrenzend zu dem Anschlag (56), jedoch von diesem axial beabstandet, angeordnet ist, so daß der Vorsprung (76), der sich von dem Skalpellgriffstück (22, 92) erstreckt, sich in dem Längsschlitz (52, 108) und über den Bereich (62) verschiebt, um in eine Halteraststellung zwischen dem Bereich (62) und dem Anschlag (56) einzuschnappen.

10. Mikrochirurgisches Skalpell (20, 90) nach Anspruch 5, worin der mindestens eine Längsschlitz (52) umfaßt:
ein Paar gegenüberliegende Längsschlitze (52), die in der Bohrung (36) auf gegenüberliegenden Seiten des Basisbereichs (32) der Abdeckung (24, 96) angeordnet sind.

11. Mikrochirurgisches Skalpell (20 90) nach Anspruch 5, worin das längliche Skalpellgriffstück (22, 92) weiter umfaßt:
mehrere parallele Umfangsnuten, die um das andere Ende (78) des Skalpellgriffstücks (22, 92) angeordnet sind, um die Handhabung des Skalpellgriffstücks (22, 92) durch einen Arzt zu erleichtern, um zuerst die Abdeckung (24, 96) mit dem Basisteil (78) des Skalpellgriffstücks (22, 92) auszurichten und mit diesem in Eingriff zu bringen und anschließend die Abdeckung (24, 96) von dem Basisbereich (78) des Skalpellgriffstücks (22, 92) zu dem Ende (70) des Griffstücks (22, 92), daß die Skalpellschneide (26, 94) hält, zu schieben.

12. Mikrochirurgisches Skalpell (20, 90) nach Anspruch 1, worin sich die Fläche jedes nachfolgenden Querschnitts an jeder Stelle weiter weg von dem Basisbereich (78) vergrößert, so daß der Haubenbereich (34, 100) eine aufgefächerte Ausbildung darstellt, um eine verlängerte und gewinkelte Ausbildung des Skalpellschneide (26, 94) bezüglich des länglichen Skalpellgriffstücks (26, 94) anzunehmen.

## Revendications

1. Ensemble de scalpel microchirurgical (20, 90) comprenant :
une poignée de scalpel allongée (22, 92) ayant un axe longitudinal par lequel la poignée (22, 92) peut être saisie par un chirurgien et tournée autour de son axe longitudinal ;
une lame de scalpel (26, 94) fixée à une extrémité (70) de ladite poignée de scalpel (22, 92) se terminant par au moins une arête coupante active ; et
un élément de protection (24, 96) relié de façon amovible à ladite extrémité (70) de ladite poignée de scalpel (22, 92) et s'étendant à partir de ladite extrémité de ladite poignée de scalpel (22, 92) servant à entourer ladite lame de scalpel (26, 94) quand l'élément de protection (24, 96) est disposé sur ladite extrémité (70) dudit scalpel, ledit élément de protection (24, 96) est conçu pour être glissé depuis la base (28) vers l'extrémité (70) de ladite poignée de scalpel (22, 92) pour être relié à l'extrémité (70) de ladite poignée de scalpel (22, 92), dans lequel ledit élément de protection (24, 96) comprend :
une partie de base (32, 98) servant à maintenir de façon amovible ladite extrémité (70) de ladite poignée de scalpel (22, 92), ladite partie de base (32, 98) ayant un alésage allongé servant à recevoir de façon coulissante l'autre extrémité (70) de ladite poignée de scalpel allongée (22, 92) et pour mettre en prise de façon amovible ladite extrémité (70) de ladite poignée de scalpel (22, 92) ; et
une partie de capot (34, 100) reliée à ladite partie de base (32, 98), et comprenant en outre un bord circonférentiel qui délimite une ouverture de la partie de capot (34, 100) ;
ladite poignée de scalpel allongée (22, 92) ayant une patte (76) qui s'étend dans le sens latéral se projetant à l'extérieur de façon radiale à partir de ladite poignée de scalpel allongée (22, 92) globalement au niveau de ladite extrémité (70) de celle-ci, caractérisée en ce que ladite lame de scalpel (26, 94) s'étend à partir de ladite poignée de scalpel (22, 92) avec un angle par rapport audit axe longitudinal de ladite poignée de scalpel (22, 92), l'axe longitudinal de ladite patte (76) et ladite lame de scalpel (26, 94) sont tous deux globalement dans le même plan, ladite partie de capot (34, 100) a une configuration agrandie par rapport à ladite partie de base (32, 98) et est espacée de ladite lame de scalpel (22, 92) et entoure celle-ci de façon pratiquement complète quand ledit élément de protection (24, 96) est en position installée, ladite partie de capot (34, 100) comprenant des surfaces allongées de parois latérales qui s'opposent mutuellement (38, 100) et des surfaces courtes de parois latérales qui s'opposent mutuellement (40) de sorte à paraître sensiblement rectangulaires à n'importe quelle coupe transversale prise le long d'une ligne perpendiculaire à un axe longitudinal central de ladite poignée de scalpel (22, 92) et
la largeur totale de la patte (76) et de la poignée de scalpel (22, 92) conjointement mesurées de façon perpendiculaire à l'axe de la poignée de scalpel (22, 92) est plus longue que la distance paralléle intérieure entre les surfaces allongées de parois latérales (38, 102) et plus courte que la distance parallèle intérieure entre les surfaces courtes de parois latérales (40) de la partie de capot (34, 100).

2. Ensemble de scalpel microchirurgical selon la revendication 1, dans lequel :
lesdites surfaces allongées de parois latérales (38, 100) sont globalement planes et ladite partie de capot comprenant en outre une partie d'étranglement adjacente à la partie de base (32, 98) de l'élément de protection, ladite partie d'étranglement étant délimitée en partie par un segment desdites surfaces courtes de parois latérales mutuellement opposées (40) qui convergent de façon radiale vers l'alésage (36, 106) de la partie de base (32, 98) dudit élément de protection (24, 96) ;

3. Ensemble de scalpel microchirurgical (20, 90) selon la revendication 2, dans lequel :
ledit segment desdites surfaces courtes de parois latérales mutuellement opposées (40) qui délimite en partie ladite partie d'étranglement dudit capot (34, 100) est arqué vers l'extérieur.

4. Ensemble de scalpel microchirurgical (20, 90) selon la revendication 2, dans lequel :
lesdites surfaces courtes de parois latérales mutuellement opposées (40) de ladite partie de capot (34, 100) sont arquées vers l'extérieur.

5. Ensemble de scalpel microchirurgical (20, 90) selon la revendication 1, dans lequel ledit élément de protection (24, 96) comprend en outre :
au moins une fente allongée (50, 108) formée à l'intérieur dudit alésage allongé (36, 106) et calibrée de sorte à coopérer avec ladite patte (76) de ladite poignée de scalpel allongée (22, 92), ladite fente allongée (50, 108) se terminant par une butée (56) pour limiter le mouvement relatif entre ledit élément de protection (24, 96) et ladite poignée de scalpel allongée (22, 92).

6. Ensemble de scalpel microchirurgical (20, 90) selon la revendication 1, dans lequel :
ladite partie de capot (34, 100) est pourvue de plusieurs ouvertures (44, 104) formées à travers ladite partie de capot (34, 100) pour faciliter la stérilisation de ladite lame de scalpel (26, 94) fixée à ladite extrémité dudit ensemble de scalpel microchirurgical (20, 90).

7. Ensemble de scalpel microchirurgical (20, 90) selon la revendication 5, dans lequel lesdites ouvertures (44, 104) comprennent :
des fentes allongées mutuellement parallèles formées à travers ledit élément de protection (24, 96) dans un emplacement actif dudit élément de protection de façon adjacente à ladite lame de scalpel (26, 94) et ayant des axes longitudinaux centraux qui sont mutuellement parallèles à l'axe longitudinal de ladite poignée de scalpel (22, 92).

8. Ensemble de scalpel microchirurgical (20, 90) selon la revendication 1, dans lequel ladite poignée de scalpel allongée (22, 92) comprend :
un élément allongé qui est essentiellement hexagonal en coupe transversale et ayant plusieurs rainures circonférentielles mutuellement parallèles (72) formées au niveau de ladite extrémité (70) de ladite poignée de scalpel (22, 92) de telle sorte que le mouvement axial dudit scalpel microchirurgical est facilité par lesdites rainures circonférentielles parallèles (72) et le mouvement latéral et de rotation du scalpel est facilité par ladite configuration hexagonale de ladite poignée de scalpel (22, 92).

9. Ensemble de scalpel microchirurgical (20, 90) selon la revendication 5 et comprenant en outre :
une partie d'appui en saillie (62) disposée à l'intérieur d'au moins une desdites fentes allongées (52, 108) de façon adjacente à ladite butée (56), mais espacée axialement de celle-ci, de telle sorte que ladite patte (76) s'étendant depuis ladite poignée de scalpel (22, 92) coulisse à travers ladite fente allongée (52, 108) et sur ladite partie (62) pour s'encliqueter dans une position de repos et de retenue entre ladite partie (62) et ladite butée (56).

10. Ensemble de scalpel microchirurgical (20, 90) selon la revendication 5, dans lequel ladite fente allongée (52) comprend :
deux fentes allongées mutuellement opposées (52) disposées à l'intérieur dudit alésage (36) sur des côtés opposés de la base (32) dudit élément de protection (24, 96).

11. Ensemble de scalpel microchirurgical (20, 90) selon la revendication 5, dans lequel ladite poignée de scalpel allongée (22, 92) comprend en outre :
plusieurs rainures circonférentielles mutuellement parallèles (80) formées autour de l'autre extrémité (78) de ladite poignée de scalpel (22, 92) pour faciliter le maniement de la poignée de scalpel (22, 92) par un médecin pour tout d'abord aligner et mettre en prise l'élément de protection (24, 96) avec la base (78) de la poignée de scalpel (22, 92) et ensuite faire glisser l'élément de protection (24, 96) depuis la base (78) de la poignée de scalpel (22, 92) vers l'extrémité (70) de la poignée (22, 92) en maintenant ladite lame de scalpel (26, 94).

12. Ensemble de scalpel microchirurgical (20, 90) selon la revendication 1, dans lequel la zone de chaque coupe transversale séquentielle va en augmentant à chaque emplacement en s'éloignant de ladite partie de base (78) de telle sorte que ladite partie de capot (34, 100) présente une configuration en éventail pour recevoir une configuration étendue et en angle de ladite lame de scalpel (26, 94) par rapport à ladite poignée de scalpel allongée (26, 94).
